(19) Europäisches Patentamt
European Patent Office
Office européen des brevets

(11) Publication number: **0 082 805**
**A2**

(12) # EUROPEAN PATENT APPLICATION

(21) Application number: 82730148.2

(22) Date of filing: 17.12.82

(51) Int. Cl.$^3$: C 07 D 457/06
A 61 K 31/48

(30) Priority: 18.12.81 DE 3150918
26.04.82 DE 3216300

(43) Date of publication of application:
29.06.83 Bulletin 83/26

(84) Designated Contracting States:
AT BE CH DE FR GB IT LI LU NL SE

(71) Applicant: SCHERING AKTIENGESELLSCHAFT Berlin und Bergkamen
Müllerstrasse 170/178 Postfach 65 03 11
D-1000 Berlin 65(DE)

(72) Inventor: Neef, Günter, Dr.
Darmstädter Strasse 9
D-1000 Berlin 15(DE)

(72) Inventor: Eder, Ulrich, Dr.
Zeltinger Strasse 17
D-1000 Berlin 28(DE)

(72) Inventor: Sauer, Gerhard, Dr.
Königsbacher Zeile 41A
D-1000 Berlin 28(DE)

(72) Inventor: Ast, Gerhard
Speerweg 47
D-1000 Berlin 28(DE)

(72) Inventor: Schröder, Gertrud, Dr.
Theodor-Franck-Strasse 3
D-1000 Berlin 42(DE)

(54) **Novel ergotanilides, their preparation and use.**

(57) Compounds of the formula

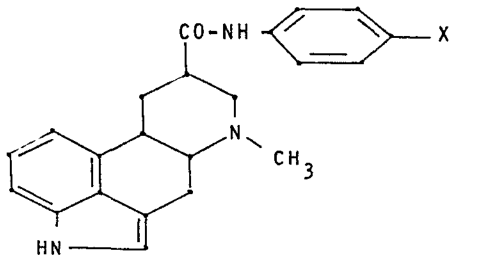

wherein
X is hydrogen, fluorine, chlorine or bromine, or a pharmacologically acceptable acid addition salt thereof are antihypotonically effective.

Moreover, ergotanilides of the formula

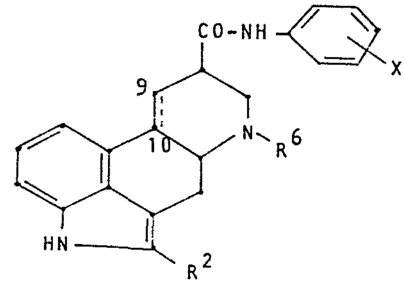

$C_9$ ===== $C_{10}$ is a C–C single or C=C double bond,
$R^2$ is hydrogen, chlorine, or bromine,
$R^6$ is $C_{1-4}$ alkyl, and
X is H, $C_{1-4}$ alkyl, $C_{1-4}$ alkoxy, fluorine, chlorine, bromine, $NH_2$, NHR' (R' = $C_{1-4}$ alkyl or $C_{1-4}$ acyl), NR'R" (R'

./...

EP 0 082 805 A2

= $C_{1-4}$ alkyl or $C_{1-4}$ acyl; R″ = $C_{1-4}$ saturated or unsaturated heterocyclic ring wherein a $CH_2$— or CH-group thereof can be substituted by N, O, or S), or SR‴ (R‴ = $C_{1-4}$ alkyl or phenyl),

can be prepared by reacting the corresponding lysergic acid methyl esters or lysergic acid ethyl esters with a corresponding dimethylaluminum anilide in an inert solvent.

# NOVEL ERGOTANILIDES, THEIR PREPARATION AND USE

## Background of the Invention

The present invention relates to new ergotanilides.

As is known, anilides of lysergic acid and of 9,10-dihydrolysergic acid are valuable pharmaceuticals. U.S. Patent 4,101,552 discloses a number of lysergic acid and 9,10-dihydrolysergic acid derivatives which have antihypertensive effect on the blood pressure. U.S. Patent 3,904,633 describes substituted anilides of lysergic acid and of 9,10-dihydrolysergic acid exhibiting antiserotonin activity, a depressive effect on the central nervous sytem, or an antidepressive effect similar to that of tricyclic antidepressants.

## Summary of the Invention

It is an object of this invention to provide new ergotanilides having valuable pharmacological properties.

It is another object of this invention to provide a process for the preparation of anilides in the ergoline series producing the desired anilides stereospecifically in a high yield.

Upon further study of the specification and appended claims, further objects and advantages of this invention will become apparent to those skilled in the art.

These objects have been attained based on the surprising finding that the compounds of Formula (I')

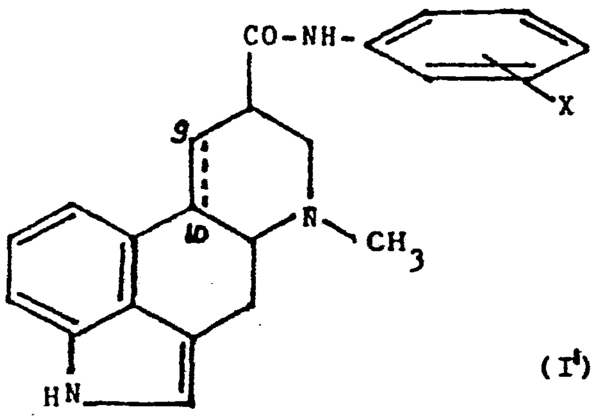

(I')

wherein

$C_9$----$C_{10}$ is a C-C single bond, and

X is hydrogen or halogen (F, Cl, Br) in the 4-position,

show antihypotonic activity. Physiologically acceptable acid addition salts thereof are also included.

These objects have also been achieved by providing a process for the preparation of ergot derivatives of Formula (I)

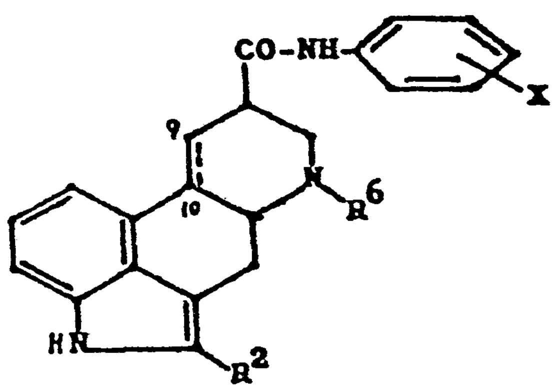

(I),

wherein

$C_9$----$C_{10}$ is a C-C single or C=C double bond,

$R^2$ is hydrogen, chlorine, or bromine,

$R^6$ is $C_{1-4}$ alkyl, and

X is $C_{1-4}$ alkyl, $C_{1-4}$ alkoxy, hydrogen, fluorine, chlorine, bromine, $NH_2$, NHR' (R' = $C_{1-4}$ alkyl or $C_{1-4}$ acyl), NR'R'' (R' = $C_{1-4}$ alkyl or $C_{1-4}$ acyl; R'' = $C_{1-4}$ alkyl, or R' and R'' together mean a 5- or 6-membered, saturated or unsaturated heterocyclic ring wherein a $CH_2$- or CH-group can be substituted by N, O, or S), or SR''' (R''' = $C_{1-4}$ alkyl or phenyl),

comprising reacting, in an inert solvent, corresponding lysergic acid esters of Formula (II)

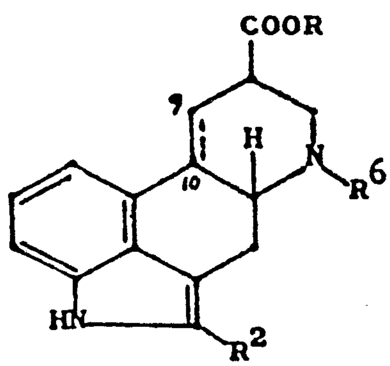

(II),

wherein

$C_9$----$C_{10}$ , $R^2$, and $R^6$ are as defined above,

and

R is methyl or ethyl,

with the reaction product from the reaction of equimolar amounts of trimethylaluminum and (a) aniline or (b) an aniline derivative of the formula

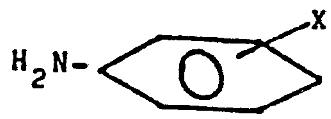

wherein X is as defined above,
thus forming an intermediate of the formula

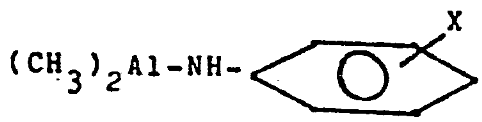

## Detailed Discussion

In particular, 6-methylergoline-8β-carboxylic acid anilide and 6-methylergoline-8β-carboxylic acid (4-fluoroanilide) show, in the test model by Timmermanns [Timmermanns, P.B.M.W.M., Kwa, H.Y., and Van Zwieten, P.A., Naunyn-Schmiedebergs Arch. Pharmacol. 310 : 189-193 (1979)] on despinalized rats, an increase of medium arterial blood pressure, of peripheral vascular resistance, and of cardiac output per minute. For the tests, normotonic male rats weighing 280-320 g were utilized. The animals were decerebrated, despinalized, subjected to bilateral vagotomy, and placed under a small-animal respirator. The compounds of this invention were administered intravenously to the animals (dose: 0.1 and 1.0 mg/kg). Blood pressure was measured live via a catheter in the A. femoralis, and the cardiac frequency was determined by a concomitantly recorded electrocardiogram [derivation A according to Spoerri, H., Arch. Wiss. Prakt. Tierheilkunde 79 : 1-57 (1944)]. The cardiac output per minute was determined according to the low-temperature dilution method (Mannesmann, G. and Mueller, B., J. Pharmacol. Meth. 4 : 11-18 (1980)].

For this purpose, a catheter was placed in the right V. jugularis of the animals to inject the refrigerated solution, and a thermistor was placed in the abdominal aorta. The peripheral vascular resistance was determined

according to the following formula:

$$\frac{6 \times P_M}{V_m \text{ (ml/min/100 g BW)}}$$

wherein

$P_M$ is the medium arterial blood pressure and
$V_m$ is the cardiac output per minute and
BW is the body weight.

The tested compounds showed, in both doses investigated, marked blood-pressure-raising effects accompanied by an increase in peripheral vascular resistance and cardiac output per minute. Upon administration of a dose of 1 mg/kg of these compounds, an increase in blood pressure lying 100% above the initial value was still present after 1/2 to 1 hour after injection. Cardiac frequency was not affected. The discovered effects can be antagonized in dependence upon the dose by $\alpha_1$-receptor blockers, such as prazosin.

The compounds of this invention are thus suitable as medicinal agents for hypotonia therapy in mammals, including humans, e.g., to raise blood pressure in analogy to the administration of the agent **norfenefrine** e.g., for treatment of **orthostatic dysregulations. Thus, the preferred compounds of the invention are** 6-**methylergoline-8ß-carboxylic acid anilide and** 6-**methylergoline-8ß-carboxylic acid (**4-**fluoroanilide).**

The compounds of this invention are utilized by following conventional methods for galenic pharmacy for the preparation of medicines preferably for oral administration. For this purpose, the compounds are used either in the free form as a base or in the form of a physiologically compatible acid addition salt in, for example, elixirs, syrups, dragees, tablets, or capsules, optionally in conjunction with conventional auxiliary agents and/or excipients. (The acid addition salts can be conventionally obtained

by reacting the free base with the corresponding acid, such as, for example, tartaric acid, maleic acid, or benzoic acid, and subsequently purifying them, e.g., by recrystallization and/or chromatography.)

The dosage of the compounds of this invention is, in case of human patients, in the range from 5 to 100 mg per day, and, typically, unit dosage forms contain 1-20 mg of active agent.

Conventional excipients are pharmaceutically acceptable organic or inorganic carrier substances suitable for parenteral or enteral application which do not deleteriously react with the active compounds. Suitable pharmaceutically acceptable carriers include but are not limited to water, salt solutions, alcohols, gum arabic, vegetable oils, polyethylene glycols, gelatine, lactose, amylose, magnesium stearate, talc, silicic acid, hydroxymethylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmotic pressure, buffers, coloring, flavoring and/or aromatic substances and the like which do not deleteriously react with the active compounds.

The present invention furthermore concerns a novel method for the preparation of the compounds of this invention of Formula (I), which latter includes novel as well as known compounds.

The conventional processes for preparation of such compounds (see ,e.g. US Patent 3,904,633; DAS 2802023; USP 4,101,552; and German Patent 659,085; all of whose disclosures are entirely incorporated by reference herein) are based on the use of lysergic acid of the formula

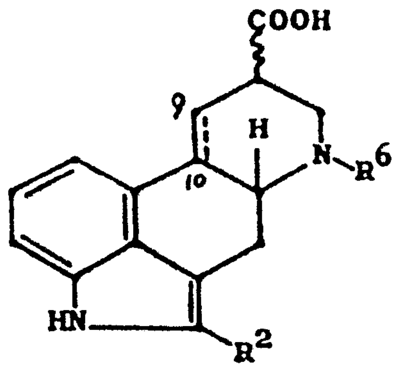

$R^6 = C_{1-4}$-Alkyl

$R^2$ = H, Br, Cl

This is first converted into a reactive, functional derivative, such as an acid chloride, acid azide, or mixed anhydride. This derivative is then reacted with a correspondingly substituted aniline to obtain the desired anilide.

The conventional processes can generally be applied well to 9,10-dihydrolysergic acid, but frequently fail in the case of lysergic acid proper, as has been determined in a comparative study by W.L. Garbrecht, J. Org. Chem. <u>24</u> : 368 (1959).

Due to the thermal and chemical instability of lysergic acid, the known methods for the preparation of lysergic acid anilides in most cases produce poor chemical yields. Even in cases where good chemical yields are obtained, reaction and/or processing conditions lead to isomerization at C-8 of the molecule's structure, resulting in mixtures of lysergic acid anilide and isolysergic acid anilide. Although separation of the isomers is generally possible, the yield of the desired product is thereby diminished.

It has now been found that the desired anilides of Formula (I) can be formed by reacting, in an inert solvent, lysergic acid esters of Formula (II)

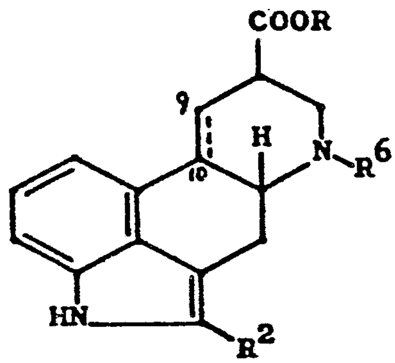

(II),

wherein

$C_9$----$C_{10}$ is a C-C single bond or a C=C double bond,

R is methyl or ethyl,

$R^2$ is hydrogen, chlorine, or bromine, and

$R^6$ is $C_{1-4}$ alkyl,

with the reaction product of the reaction of equimolar amounts of trimethylaluminum and (a) aniline, or (b) an aniline derivative of the formula

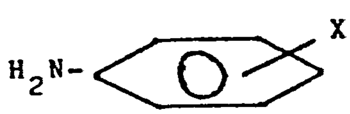

wherein X is $C_{1-4}$ alkyl, $C_{1-4}$ alkoxy, hydrogen, fluorine, chlorine, bromine, $NH_2$, NHR' (R' = $C_{1-4}$ alkyl or $C_{1-4}$ acyl), NR'R'' (R' = $C_{1-4}$ alkyl or $C_{1-4}$ acyl; R'' = $C_{1-4}$ alkyl; or R' and R'' together form a 5- or 6-membered, saturated or unsaturated heterocyclic ring (containing one N atom and the remainder C-atoms) wherein a $CH_2$- or CH-group can be substituted by N, O, or S), or SR''' (R''' = $C_{1-4}$ alkyl or phenyl), thereby forming an intermediate of the formula

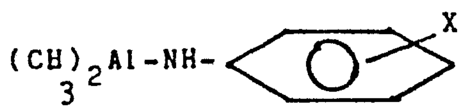

wherein X is as defined above.

In these formulae, alkyl or alkoxy of 1-4 carbon atoms is derived from alkanes, such as methane, ethane, propane, isopropane, and butane. Acyl of 1-4 carbon atoms is generally derived from aliphatic carboxylic acids, such as, for example, acetic acid, propionic acid, butyric acid, and valeric acid. When the substituents R' and R'' together form a saturated or unsaturated ring, the resultant rings are, for example, pyrrolyl, pyrrolidinyl, pyridyl, and piperidyl. In the case of substitution of a CH- or $CH_2$-group by an O, S, or N atom, resultant groups include, for example, imidazolyl, pyrazolyl, pyrazinyl, imidazolidinyl, imidazolinyl, pyrazolidinyl, pyrazolinyl, piperazinyl, morpholinyl, and isothiazolidinyl.

The reaction with the compound of Formula (II) is conducted in a temperature range of 0-40°C, preferably at room temperature, usual for 1 - 5 hours; using approximately stoichiometric amounts. The preceding reaction with trimethylaluminum is carried out at -10 to +10 °C, typically at 0°C, for 10 - 60 minutes,

again using approximately stoichiometric amounts.

The process of this invention is conducted by reacting trimethylaluminum, in an inert solvent while cooling, with the aniline or the aniline derivative, and thereafter adding the lysergic acid ester gradually. Suitable solvents, besides toluene, include benzene, hexane, and methylene chloride. It is advantageous to work under a protective gas atmosphere, such as, for example, under a nitrogen or argon atmosphere.

After conducting the reaction according to the process of this invention, the reaction mixture is worked up, thus selectively obtaining the free base or a salt of the free base. An especially suitable salt is the salt of tartaric acid. However, other acids are likewise suitable, for example, phosphorous acids, hydrochloric acid, acetic acid, benzenesulfonic acid, maleic acid, etc.

Advantages of the process of this invention include the simple nature of the reaction and working up procedures, the high chemical yields, and, above all, the surprising fact that no isomerization occurs in the reaction of the lysergic acid esters, i.e., there is no formation of isolysergic acid anilides as undesirable by-products.

The latter circumstance is nowise self-evident, if one considers that trimethylaluminum and derivatives obtained therefrom are employed as highly effective Lewis acids. The Lewis acid character of the compound of Formula (II) hardly admits of the expectation that the reaction of lysergic acid esters would take place with preservation of configuration. Also the high chemical yields are surprising to those skilled in the art since the ergoline skeleton includes additional groups (N-1, C-3, N-6) which could enter into an interaction with a Lewis acid.

All of the starting materials of the process

of this invention are either known or fully conventionally preparable.

All of the products of formula (I) of this invention are pharmacologically active per se (see foregoing and above-cited references or can be used to prepare other products of this invention or other ergoline derivatives which are of known pharmacological activity.

The process of the present invention permits to obtain, e.g. N-9,10-dihydrolysergyl-m-amino-benzoic acid diethylamide for the treatment of vascular deseases (see German Offenlegungsschrift No. 2 802 023), e.g. lysergic acid-(2'-fluoro-4'-methyl-phenyl)-amide bimaleate with antihypertensive and antiserotonin effect and exerting an action on the central nervous system (see U.S.P. 4,101,552), or, e.g. 1-methyl-9,10-dihydro-d-lysergic acid-(4-methoxy)-anilide possessing antiserotonin and central depressing activities (see U.S.P. 3,904,633).

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following example(s), all temperatures are set forth uncorrected in degrees Celsius; unless otherwise indicated, all parts and percentages are by weight.

Example

Under ice water cooling and an argon gas atmosphere, 1.83 ml of 4-fluoroaniline in 10 ml of toluene is added dropwise to 16.7 ml of a 10% strength solution of trimethylaluminum (19.2 mmol) in toluene. The mixture is

then stirred for 30 minutes at room temperature. Thereafter, a solution of 2.72 g (9.6 mmol) of lysergic acid methyl ester in 20 ml of toluene and 20 ml of methylene chloride is added dropwise to the reaction mixture. The reaction solution is then stirred for another 1.5 hours at room temperature.

The reaction mixture can be worked up in two ways.

Version A:

Under ice water cooling, excess reagent is destroyed by the dropwise addition of 10 ml of water. The reaction solution is thereafter diluted with 50 ml of methylene chloride and 50 ml of methanol, filtered over "Celite", and the filtrate is concentrated. After recrystallization of the residue from ethyl acetate/diisopropyl ether, 3.35 g of lysergic acid p-fluoroanilide is obtained (96.2%), mp 203-206° C (decomposition).

Version B:

At room temperature, 8.5 g of solid sodium fluoride is added in incremental portions to the reaction solution; the latter is stirred for another 30 minutes, filtered over "Celite", the filter residue washed with methylene chloride, and the filtrate concentrated. The resultant crude product is dissolved in about 20 ml of methanol, combined with 1.0 g of l(+)-tartaric acid, and left to crystallize overnight at -5° C. Yield: 4.4 g

(89.2%) of lysergic acid p-fluoroanilide as the hydrogen tartrate, mp 152-155° C (decomposition).

The following compounds are produced analogously:

(a) lysergic acid (3-fluoroanilide), hydrogen tartrate, mp 144-149° C (decomposition), yield 91%

(b) lysergic acid (4-methoxyanilide), hydrogen tartrate, mp 155-158° C (decomposition), yield 100%

(c) 6-methylergoline-8β-carboxylic acid (4-methylanilide), mp 241-244° C, yield 95%

(d) 6-methylergoline-8β-carboxylic acid (2-aminoanilide), mp 249-253° C, yield 85%

(e) 6-methylergoline-8β-carboxylic acid anilide, mp 231° C, yield 100%

(f) 6-methylergoline-8β-carboxylic acid (2,6-dichloroanilide), mp 262° C (decomposition), yield 86%

(g) 6-methylergoline-8β-carboxylic acid (4-fluoroanilide), mp 251-255° C, yield 98%

(h) 6-methylergoline-8β-carboxylic acid (4-chloroanilide), mp 247-251° C, yield 100%

(i) N-9,10-dihydrolysergyl-m-aminobenzoic acid diethylamide, mp 138-141° C, yield 97%

(j) 6-methylergoline-8β-carboxylic acid (3-trifluoromethylanilide), mp 130° C, yield 88%

(k) 9,10-didehydro-6-n-propyl-8β-ergolinecarboxylic acid (4-fluoroanilide), hydrogen tartrate, mp 138-142° C. (decomposition), yield 92%

(l) 2-chlorolysergic acid (4-methoxyanilide), hydrogen tartrate, mp 146-148° C, yield 87%.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

WHAT IS CLAIMED IS:

1. A compound of the formula

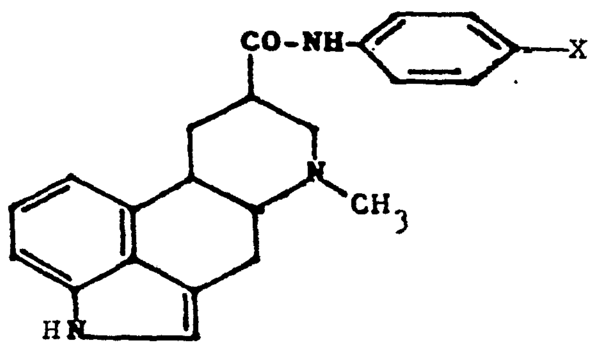

wherein

X is hydrogen, fluorine, chlorine or bromine, or a pharmacologically acceptable acid addition salt thereof.

2. 6-methylergoline-8β-carboxylic acid anilide, a compound of claim 1.

3. 6-methylergoline-8β-carboxylic acid (4-fluoroanilide), a compound of claim 1.

4. A compound of claim 1 which is in the form of a tartrate salt.

5. A compound of claim 1 wherein X is Cl.

6. A compound of claim 1 wherein X is Br.

7. A pharmaceutical composition comprising an amount of a compound of claim 1 effective to raise blood pressure and a pharmaceutically acceptable carrier.

8. A pharmaceutical composition comprising an amount of a compound of claim 2 effective to raise blood pressure and a pharmaceutically acceptable carrier.

9. A pharmaceutical composition comprising an amount of a compound of claim 3 effective to raise blood pressure and a pharmaceutically acceptable carrier.

10. A method of raising blood pressure in a patient in need of such treatment comprising administering an amount of a compound of claim 1 effective to raise the blood pressure of the patient.

11. A method of raising blood pressure in a patient in need of such treatment comprising administering an amount of a compound of claim 2 effective to raise the blood pressure of the patient.

12. A method of raising blood pressure in a patient in need of such treatment comprising administering an amount of a compound of claim 3 effective to raise the blood pressure of the patient.

13. A process for the preparation of an ergot derivative of the formula

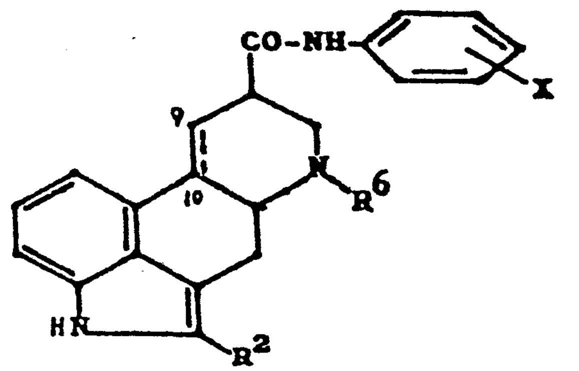

wherein

$C_9$----$C_{10}$ is a C-C single or C=C double bond,

$R^2$ is hydrogen, chlorine, or bromine,

$R^6$ is $C_{1-4}$ alkyl, and

X is $C_{1-4}$ alkyl, $C_{1-4}$ alkoxy, hydrogen, fluorine, chlorine, bromine, $NH_2$, NHR', NR'R'' or SR''', wherein R' is $C_{1-4}$ alkyl or $C_{1-4}$ alkanoyl, R'' is $C_{1-4}$ alkyl, or R' and R'' together with the common N-atom form a 5- or 6-membered, saturated or unsaturated heterocyclic ring containing one N-atom, the remainder being C-atoms or form such a ring wherein a $CH_2$- or CH-group is replaced by N, O, or S, and R''' is $C_{1-4}$ alkyl or phenyl,

comprising reacting, in an inert solvent, a corresponding lysergic acid ester of the formula

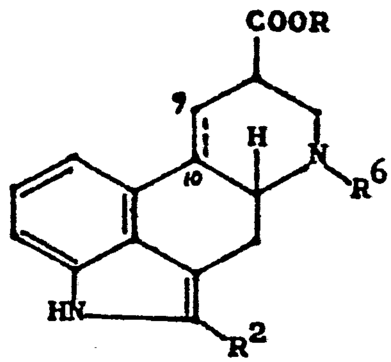

wherein

R is methyl or ethyl,

with a compound of the formula

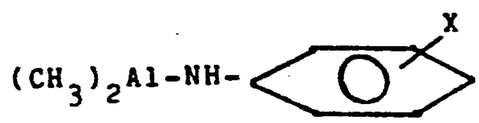 .

14. A process of claim 13 wherein the lysergic acid ester is reacted with the reaction product from the reaction of substantially equimolar amounts of trimethylaluminum and an aniline compound of the formula

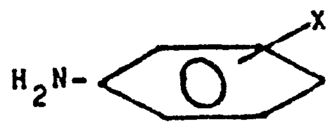

which product comprises an intermediate of the formula

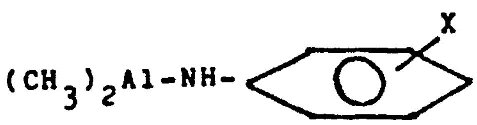

15. A process of claim 13 wherein the reaction with the lysergic acid ester is carried out at 0-40°C.

16. A process of claim 14 wherein the reaction with the lysergic acid ester is carried out at 0-40°C; the preceding reaction with trimethylaluminum is carried out at -10 - +10 °C; and both reactions are conducted in toluene, benzene, hexane or methylene chloride and under an inert gas atmosphere.